(19) Europäisches Patentamt
European Patent Office
Office européen des brevets

(11) Veröffentlichungsnummer: **0 256 262 A1**

# (12) EUROPÄISCHE PATENTANMELDUNG

(21) Anmeldenummer: **87109164.1**

(22) Anmeldetag: **25.06.87**

(51) Int. Cl.[4]: **A61K 7/48**, A61K 7/06, C01B 15/037

Die Bezeichnung der Erfindung wurde geändert (Richtlinien für die Prüfung im EPA, A-III, 7.3).

(30) Priorität: **15.07.86 DE 3623826**

(43) Veröffentlichungstag der Anmeldung: **24.02.88 Patentblatt 88/08**

(84) Benannte Vertragsstaaten: **AT BE CH DE ES FR GB IT LI NL**

(71) Anmelder: **Wella Aktiengesellschaft**
**Berliner Allee 65**
**D-6100 Darmstadt(DE)**

(72) Erfinder: **Hartmann, Peter**
**Hoffmannstrasse 6**
**D-6100 Darmstadt(DE)**
Erfinder: **Köhler, Joachim, Dr.**
**Im Trappengrund 1A**
**D-6107 Reinheim(DE)**

(54) **Verwendung von alpha-Bisabolol zur Stabilisierung von Wasserstoffperoxid.**

(57) Kosmetisches Mittel mit einem Gehalt an Wasserstoffperoxid und alpha-Bisabolol der Formel (I)

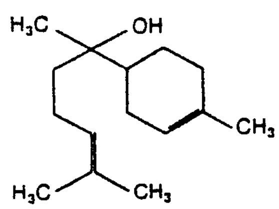

(I)

sowie die Verwendung von (I) zur Stabilisierung von Wasserstoffperoxid.

EP 0 256 262 A1

**Kosmetische Mittel mit einem Gehalt an Wasserstoffperoxid und alpha-Bisabolol sowie die Verwendung von alpha-Bisabolol zur Stabilisierung von Wasserstoffperoxid**

Gegenstand der Erfindung sind kosmetische Mittel mit einem Gehalt an Wasserstoffperoxid und alpha-Bisabolol der Formel (I)

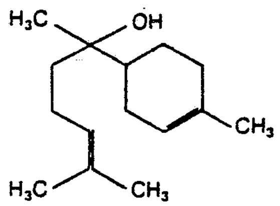

(I)

sowie die Verwendung von alpha-Bisabolol zur Stabilisierung von Wasserstoffperoxid.

In kosmetischen Mitteln wird Wasserstoffperoxid beispielsweise zur Desinfektion der Haut, zur oxidativen Färbung und Blondierung von Haaren und zur oxidativen Nachbehandlung nach einer reduktiven Haarverformung eingesetzt.

Wasserstoffperoxid ist eine metastabile Verbindung und zerfällt unter Sauerstoffentwicklung. Sauerstoff besitzt eine sehr geringe Löslichkeit in Wasser (Bunsenscher Absorptionskoeffizient bei 20 Grad Celsius: 0,03). In geschlossenen Gefäßen kann deshalb - insbesondere bei längerer Lagerzeit - ein Überdruck entstehen. Als Folge davon kann ein Teil des im Gefäß enthaltenen Mittels überschäumen oder verspritzen. Weiterhin kann der Überdruck zum Bruch des Gefäßes sowie bei Verwendung von Kunststoffgefäßen zur Deformationen oder sogar zu Bombagen führen.

In wasserstoffperoxidhaltigen kosmetischen Mitteln muß das Wasserstoffperoxid deshalb stabilisiert werden.

In der Literatur W. Machu "Das Wasserstoffperoxid", Springer-Verlag (1951), Seiten 197 - 208 ist eine Vielzahl an Stabilisatoren für Wasserstoffperoxid, unter anderem auch bestimmte organische Verbindungen, beschrieben. Diese werden jedoch bezüglich ihrer Stabilisierungsfähigkeit über einen längeren Zeitraum hinweg als nicht völlig zufriedenstellend beurteilt.

Aus dem deutschen Reichspatent 91 285 (C. Raspe) ist bekannt, daß unter anderem die Terpene Thymol, Menthol und Campher wasserstoffperoxidhaltige Lösungen zu stabilisieren vermögen. Diese Verbindungen weisen jedoch einen unangenehmen scharfen Geruch auf und sind für kosmetische Zwecke nur sehr beschränkt verwendbar.

Aufgabe der Erfindung ist es daher, ein kosmetisches Mittel mit einem Gehalt an Wasserstoffperoxid zur Verfügung zu stellen, worin das Wasserstoffperoxid besser stabilisiert ist als bei der Verwendung vergleichbarer bekannter Stabilisatoren und das keinen unangenehmen Geruch aufweist.

Hierzu wurde nun gefunden, daß durch die Verwendung von alpha-Bisabolol der Formel (I)

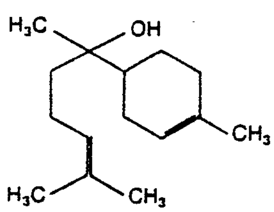

(I)

als Stabilisator von Wasserstoffperoxid, die gestellte Aufgabe in hervorragender Weise gelöst wird.

Obgleich das alpha-Bisabolol auch ein Terpen darstellt, ist seine Fähigkeit Wasserstoffperoxid zu stabilisieren überraschenderwese 5 - 10mal größer als die von Thymol, Menthol oder Campher. Diese Eigenschaft, welche nicht vorhersehbar war, wird durch den Vergleichsversuch in Beispiel 1 belegt.

Zudem besitzt das alpha-Bisabolol einen schwach süßlichen, blumigen Geruch, wodurch keine unangenehme geruchliche Belastung der kosmetischen Mittel auftritt.

Ferner wurde festgestellt, daß die Rötungen der Haut und Brennerscheinungen, welche beim Einsatz von wasserstoffperoxidhaltigen Mitteln auftreten können, durch das alpha-Bisabolol vermindert werden.

Gegenstand der Erfindung ist somit weiterhin ein kosmetisches Mittel, welches Wasserstoffperoxid und alpha-Bisabolol der Formel (I) enthält.

In dem neuen kosmetischen Mittel soll das Wasserstoffperoxid in einer Menge von etwa 0,5 bis etwa 18 Gewichtsprozent enthalten sein, während die Menge an alpha-Bisabolol etwa 0,01 bis etwa 2,5 Gewichtsprozent beträgt.

Der pH-Wert des neuen Mittels liegt vorzugsweise im Bereich von etwa 1,5 bis 3,5.

In dem erfindungsgemäßen Mittel kann das alpha-Bisabolol als alleiniger Stabilisator oder in Kombination mit anderen bereits bekannten Stabilisatoren enthalten sein.

Als bekannte Stabilisatoren kommen beispielsweise aromatische Sulfonsäuren oder aber saure Salze starker Säuren, wie zum Beispiel Alkalihydrogensulfate, saure Alkalipyrophosphate, Natriummetaphosphat und Alkalipolyphosphate in Betracht. Weiterhin können Ascorbinsäure, Schwefelsäure, Phosphorsäure, Pyro-oder Polyphosphorsäuren, Salzsäure, Oxalsäure, Benzoesäure, Salicylsäure, Malonsäure, Zitronensäure und Gerbsäuren sowie Phenol, Thymol, Paraformaldehyd, 4-Acetamido-phenol und Phenacetin enthalten sein.

In dem neuen Mittel können ebenfalls Tenside enthalten sein, unter der Voraussetzung, daß deren hydrophobe Anteile gesättigt und nicht oxidierbar sind.

Als geeignete Tenside kommen vor allem

a) anionische oberflächenaktive Agenzien, wie beispielsweise Alkali-, Erdalkali-, Ammonium-oder Alkanolaminsalze von Alkylsulfonaten, Alkylsulfaten und Alkylethersulfaten, wie zum Beispiel Natriumlaurylalkoholdiglykolethersulfat, Natrium-oder Triethanolaminsalze von Alkylsulfaten mit 12 bis 18, vorzugsweise 12 bis 14 Kohlenstoffatomen, die Natrium-oder Triethanolaminsalze von Lauryl-oder Tetradecylethersulfaten, das Dinatriumsalz des Sulfosuccinhalbesters von Alkanolamiden, Seifen und Polyethercarbonsäuren, sowie

b) nichtionische oberflächenaktive Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-und Stearylalkohol, allein oder im Gemisch, Fettalkohole von oxethyliertem Lanolin oder oxethyliertes Lanolin, Polyglycolether von Alkylphenolen mit 8 bis 30 Kohlenstoffatomen im Alkylrest und 1 bis 10 Glycoleinheiten im Molekül, Fettsäurealkanolamide sowie oxethylierte Sorbitanfettsäureester, weiterhin

c) kationische oberflächenaktive Agenzien, wie beispielsweise das Dilauryldimethylammoniumchlorid, die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl-oder Cetylpyridiniumchlorid, Alkylamidethyltrimethylammoniumethersulfate, Verbindungen mit kationischem Charakter wie Aminoxide, beispielsweise Alkyldimethylaminoxide oder Alkylaminoethyldimethylaminoxide und

d) amphotere oder zwitterionische oberflächenaktive Agenzien wie beispielsweise Carboxylderivate von Imidazol, N-Alkyl-und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureaminopropyldimethylaminoessigsäurebetain, in Betracht.

Zur Wirkungssteigerung der wasserstoffperoxidhaltigen Oxidationsmittel für Haarbehandlungsprodukte können Quell-und Penetrationsstoffe, wie zum Beispiel Harnstoff, 2-Pyrrolidon, 1-Methyl-2-pyrrolidon und Dipropylenglycolmonomethylether, zugesetzt werden.

Selbstverständlich kann das erfindungsgemäße Mittel weitere für kosmetische Mittel übliche und bekannte Zusatzstoffe, zum Beispiel Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl, ferner Farbstoffe, Trübungsmittel, wie zum Beispiel Polyethylenglykolester, oder Alkohole, wie beispielsweise Ethanol, Propanol und Isopropanol, Lösungsvermittler, Stabilisatoren, Puffersubstanzen, Parfümöle, haarkonditionierende bzw. haarpflegende Bestandteile, wie zum Beispiel Lanolinderivate, Cholesterin oder Betain, enthalten. Die erwähnten Bestandteile werden in den für solche Zwecke üblichen Mengen verwendet, zum Beispiel die Verdickungsmittel in einer Menge von etwa 0,1 bis 25 Gewichtsprozent.

Die Zubereitungsform kann beispielsweise eine Lösung, insbesondere eine wäßrige oder wäßrig-alkoholische Lösung, eine Creme, ein Gel, eine Paste oder eine Emulsion sein.

Es ist ebenfalls möglich, die neuen kosmetischen Mittel unter Druck in Aerosoldosen abzufüllen und diese daraus in Form eines Schaumes zu entnehmen.

Das erfindungsgemäße Mittel wird vorzugsweise als Oxidationsmittel für die Haarfärbung oder zur oxidativen Nachbehandlung nach einer reduktiven Haarverformung eingesetzt.

Im Falle der Haarfärbung wird das neue Oxidationsmittel unmittelbar vor dem Gebrauch mit einer farbstoffhaltigen Trägermasse vermischt und eine für die Haarfärbung ausreichende Menge des Gemisches auf das Haar aufgetragen. Nach einer Einwirkungszeit von etwa 10 bis 45 Minuten bei etwa 15 bis 50 Grad Celsius wird das Haar mit Wasser gespült, getrocknet und gegebenenfalls mit einer schwachen physiologisch verträglichen organischen Säure, wie zum Beispiel Zitronensäure oder Weinsäure, nachgespült.

Bei der Haarverformung wird zunächst das Haar mit einem Reduktionsmittel, beispielsweise mit einer alkalischen Lösung eines mercaptocarbonsauren Salzes, behandelt und in die gewünschte Form, beispielsweise durch Verwendung von Wicklern oder durch Auskämmen, gebracht. Nach einer ausreichenden Einwirkungszeit wird das Haar mit Wasser gespült, mit dem neuen Oxidationsmittel nachbehandelt, wiederum mit Wasser gespült und gegebenenfalls mittels einer Wasserwellung zur Frisur gelegt.

Die nachfolgenden Beispiele sollen den Gegenstand der Erfindung näher erläutern.

**Beispiele für kosmetische Mittel**

**Beispiel 1:** Oxidationsmittel mit einem pH-Wert von 2,1 für die Haarfärbung oder Haarbleichung

| | |
|---|---|
| 0,5 g | alpha-Bisabolol |
| 12,0 g | Wasserstoffperoxid, 50-prozentige wäßrige Lösung |
| 2,0 g | Cetylstearylalkohol |
| 1,0 g | Wollwachs |
| 0,5 g | Natriumlaurylsulfat |
| 0,5 g | 2-Octyl-decanol |
| 1,0 g | Nonylphenol, mit 10 Mol Ethylenoxid oxethyliert |
| 0,2 g | Parfümöl |
| 0,1 g | o-Phosphorsäure, 85-prozentige wäßrige Lösung |
| 82,2 g | Wasser, vollentsalzt |
| 100,0 g | |

Für den Vergleichsversuch wurde in dem obigen Mittel (1) das alpha-Bisabol mengengleich durch Thymol (1a), Menthol (1b) und Campher (1c) ersetzt oder im Fall (1d) der Stabilisator ganz weggelassen.

Die 3monatige Lagerung bei 40 Grad Celsius sowie die 6monatige Lagerung bei 30 Grad Celsius der zu vergleichenden Mittel in Polyethylenflaschen erbrachten die gleichen Ergebnisse. Sie sind in der Tabelle 1 aufgeführt

Tabelle 1:

| Mittel | (1) | (1a) | (1b) | (1c) | (1d) |
|---|---|---|---|---|---|
| Druck | - | - | + | + | ++ |
| Verlust an Wasserstoffperoxid in Prozent (relativ zum Anfangsgehalt) | 0,5 | 2,5 | 5 | 3 | 11 |

Hierbei bedeutet - = kein Überdruck
\+ = Überdruck
++ = Starker Überdruck

Der Verlust an Wasserstoffperoxid wurde durch Titration mit Thiosulfat/Jod bestimmt.

Der vorstehende Vergleichsversuch zeigt die gegenüber Thymol, Menthol und Campher wesentlich bessere stabilisierende Wirkung des alpha-Bisabolols für Wasserstoffperoxid.

**Beispiel 2:** Mittel mit einem pH-Wert von 2,5 zur oxidativen Nachbehandlung nach einer reduktiven Dauerwellung

| | |
|---|---|
| 6,0 g | Natriumlaurylethersulfat, mit 2 Mol Ethylenoxid pro Mol Laurylalkohol oxethyliert |
| 0,1 g | alpha-Bisabolol |
| 5,0 g | Wasserstoffperoxid, 50-prozentige wäßrige Lösung |
| 0,1 g | Hippursäure |
| 1,0 g | Nonylphenol, mit 10 Mol Ethylenoxid oxethyliert |
| 1,0 g | Zitronensäure |
| 86,8 g | Wasser, vollentsalzt |
| 100,0 g | |

Nach 3 beziehungsweise 6monatiger Lagerung wurde kein Überdruck festgestellt.

**Beispiel 3:** Mittel mit einem pH-Wert von 2,8 zur oxidativen Nachbehandlung nach einer reduktiven Dauerwellung

| | | |
|---|---|---|
| 0,01 | g | alpha-Bisabolol |
| 4,0 | g | Wasserstoffperoxid, 50-prozentige wäßrige Lösung |
| 2,0 | g | Fettsäureamidopropyldimethylaminoessigsäure-betain, 30-prozentige wäßrige Lösung |
| 0,5 | g | Lauryldimethylaminoxid |
| 0,2 | g | Ethylendiamintetraessigsäure |
| 0,05 | g | Phenacetin |
| 0,2 | g | Natriumdihydrogenphosphat |
| 93,0 | g | Wasser, vollentsalzt |
| 100,0 | g | |

Nach 3 beziehungsweise 6monatiger Lagerung wurde kein Überdruck festgestellt.

**Beispiel 4:** Oxidationsmittel mit einem pH-Wert von 2,2 für die Haarfärbung oder Haarbleichung

| | | |
|---|---|---|
| 2,5 | g | alpha-Bisabolol |
| 24,0 | g | Wasserstoffperoxid, 50-prozentige wäßrige Lösung |
| 3,0 | g | Nonylphenol, mit 10 Mol Ethylenoxid oxethyliert |
| 0,1 | g | o-Phosphorsäure, 85-prozentige wäßrige Lösung |
| 70,4 | g | Wasser, vollentsalzt |
| 100,0 | g | |

Nach 3 beziehungsweise 6monatiger Lagerung wurde kein Überdruck festgestellt.

**Beispiel 5:** Mittel mit einem pH-Wert von 2,5 zur oxidativen Nachbehandlung nach einer reduktiven Haarentkräuselung

| | |
|---|---|
| 0,5 g | alpha-Bisabolol |
| 4,0 g | Wasserstoffperoxid, 50-prozentige wäßrige Lösung |
| 2,0 g | Kokosfettsäuremonoethanolamid |
| 10,0 g | Laurylalkohol-diglykolethersulfat-Natrium-salz, 28-prozentige wäßrige Lösung |
| 0,5 g | Parfümöl |
| 83,0 g | Wasser, vollentsalzt |
| 100,0 g | |

Nach 3 beziehungsweise 6monatiger Lagerung wurde kein Überdruck festgestellt.

Alle Prozentangaben in dieser Anmeldung stellen, soweit nicht anders angegeben, Gewichtsprozente dar.

## Ansprüche

1. Kosmetisches Mittel, dadurch gekennzeichnet, daß es Wasserstoffperoxid und alpha-Bisabolol enthält.

2. Mittel nach Anspruch 1, dadurch gekennzeichnet, daß das Wasserstoffperoxid in einer Menge von 0,5 bis 18 Gewichtsprozent enthalten ist.

3. Mittel nach den Ansprüchen 1 und 2, dadurch gekennzeichnet, daß das alpha-Bisabolol in einer Menge von 0,01 bis 2,5 Gewichtsprozent enthalten ist.

4. Mittel nach den Ansprüchen 1 bis 3, dadurch gekennzeichnet, daß der pH-Wert 1,5 bis 3,5 beträgt.

5. Verwendung von alpha-Bisabolol für die Stabilisierung von Wasserstoffperoxid.

Europäisches Patentamt

# EUROPÄISCHER RECHERCHENBERICHT

Nummer der Anmeldung

EP 87 10 9164

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 130 486 (L'OREAL)<br>* Insgesamt *<br>--- | 1-3 | A 61 K 7/48<br>A 61 K 7/06<br>C 01 B 15/037 |
| Y | PARFUMS, COSMETIQUES, AROMES, Nr. 57, Juni-Juli 1984, Seiten 55-57, Paris, FR; JELLINEK: "Alpha-bisabolol - un agent anti-inflammatoire pour produits cosmétique"<br>* Seiten 55-57 *<br>--- | 1-3 | |
| Y | FR-A- 741 469 (CHEMICA GmbH)<br>* Seite 2, linke Spalte, Zeile 27 - rechte Spalte, Zeile 40 *<br>--- | 1-3 | |
| A | F. WINTER: "Handbuch der gesammten Parfümerie und Kosmetik", 6. Auflage, Seiten 641-642, Kapitel 30; "Haarentfärbungsmittel (Blondierungsmittel)"<br>* Seite 642, "Kamillentinktur" *<br>----- | 1-5 | |

RECHERCHIERTE SACHGEBIETE (Int. Cl.4)

A 61 K
C 01 B

Der vorliegende Recherchenbericht wurde für alle Patentansprüche erstellt.

| Recherchenort | Abschlußdatum der Recherche | Prüfer |
|---|---|---|
| DEN HAAG | 29-09-1987 | FISCHER J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82